# EUROPEAN PATENT APPLICATION

(11) **EP 1 508 791 A1**
(43) Date of publication of application: **23.02.2005**
(21) Application number: 03405614.3
(22) Date of filing: 22.08.2003
(51) Int. Cl.: G01N 1/18, C12M 1/26

(54) **Device for automated bioreactor sampling**

(71) Applicant: Ismatec SA, Laboratoriumstechnik, 8152 Glattbrugg (CH)
(72) Inventor: Cannizzaro, Christopher, Swampscott, MA 01907 (US)
(74) Representative: Bremi, Tobias, Dr.

(57) **Abstract**

The invention relates to a device for automated bioreactor sampling connected to a reactor (1) comprising a sampler (4), at least one tube (21,22) fluidly connecting said reactor (1) with said sampler (4) and a pump (9) for pumping fluids from said reactor (1) to said sampler (4) and vice versa. Particularly simple and essentially dead volume-free sampling can be achieved by providing means (3,14) for flushing at least parts of the tube (21,22) with gas/sterile air between individual sampling steps. The invention furthermore relates to a method of operation of such a device.

## Description

### TECHNICAL FIELD

The invention relates to a device for automated bioreactor sampling to be connected to a reactor, comprising a sampler, at least one tube fluidly connecting said reactor with said sampler and a pump for pumping fluids from said reactor to said sampler and vice versa. The invention furthermore relates to a method for operating such a device connected to a bioreactor for sampling.

### BACKGROUND OF THE INVENTION

Analysis of chemical compounds during bioprocesses can be performed off-line, on-line or by in situ sensors. For each mode of analysis an adequate sampling system is required:
(i) for in situ analysis the analytical probe is directly inserted in the bioreactor, the membrane which separates the electrode from the broth acting as the sampling point,
(ii) for on-line analysis samples have to be pumped to the analyzer in one of two modes, continuously or discontinuously, dependent on the bioprocess monitored and the analysis system employed. The analysis frequency attainable is high compared to off-line analysis. Therefore, the sample volumes should be as low as possible.
(iii) different sampling procedures exist for off-line analysis, sampling of fermenter broth is performed manually via steam sterilizable bottom harvesting valves or by hooded samplers, whereas volatile compounds are collected from the exhaust or head-space gas,

A sampler for on-line or off-line analysis must fulfil at least two functions: (i) transport of the analyte of interest to the sensor in (a) whole culture flow, (b) flow of filtered broth, (c) a flow of buffer after diffusion of the analyte from the culture broth to the buffer; and (ii) maintain the sterile integrity of the bioreactor.

Quantitative bioreactor experiments in case of off-line analysis require sample collection at a high rate and in a reproducible manner. Since experiments are often of long duration (from days to months), methods to automate the process are invaluable. A quite thorough review of sampling and semi-on-line analysis techniques can be found in Christensen et al. (Christensen, L H., Marcher, J., Schulze, U., Carlsen, M., Min, R. W., Nielsen, J. and Villadsen, J., 1996, Semi-on-line analysis for fast and precise monitoring of bioreaction processes, Biotechnology and Bioengineering 52, 237-247) where the minimum requirements for automatic retrieval of medium samples were established: (1) device should establish reliable aseptic barrier, (2) turnover of sample components due to cellular activity should be minimized, i.e. effective quenching of metabolism, (3) device should be robust enough to allow continuous sampling over several hundred hours.

Direct sampling: The exact nature of sampling procedure will, of course, depend on objectives desired. For quantification of intracellular metabolites and/or co-factors (i.e. nucleotides, sugar phosphates) metabolic activity must be rapidly quenched (<0.2 s). Quantification of extracellular metabolite concentrations in the mg/l range will also necessitate rapid sample quenching as shown by Larson et al. (Larsson, G. and Tornkvist, M., 1996, Rapid sampling, cell inactivation and evaluation of low extracellular glucose concentrations during fed-batch cultivation, Journal of Biotechnology Vol 49, 69-82) for E. coli and S. cerevisiae fed-batch cultivations. However, often, and above all for industrial processes, the simplicity and robustness of the method is more important than absolute accuracy.

Membrane based sampling: Off-line analysis of metabolites is acceptable for monitoring, but on-line analysis is necessary for control applications. A key problem in the on-line analysis of fermentation broth is to obtain a cell-free sample since this is what most analysis techniques require for several reasons:
(i) If the sample flow is not cell-free the transport tubings are likely to act as continuous tube reactors where biological reactions during transport can change the sample's composition, which emphasises the importance of fast sample transport from the reactor to the sensor if this mode of operation is used,
(ii) cells may interact with the reagents used for analysis or directly with the transducer,
(iii) the presence of cells will reduce the lifetime of the analysis system due to adsorption.

Even if the sample is almost cell-free, but non-sterile, cells are likely to grow and attach to the inner surfaces of the tubing during transport. This microbial film can cause considerable changes during transport of the medium through the tubing.

In situ and ex situ filtration devices are available, however they often become fouled rather quickly. It is therefore sometimes concluded that such a membrane system is suitable for batch culture, but becomes rapidly fouled in continuous culture (after 5 days glucose concentrations generally start to deviate). Similar problems occur with high-density fed-batch cultures. In addition, these systems act as a cell recycle device, which complicates culture dynamics. The filters are also not suitable for all microorganisms. For example, the ABC module available on the market (Advance Biotechnology Corp., Germany), proves not useful for P. rhodozyma yeast fermentation due to secreted components that rapidly foul the membrane. Upon removal of the membrane from reactor, even after a batch reaction, the membrane is often covered in an orange/red gelatinous layer. Experiments with Escherichia coli bacteria found that after 5-7 days, there often occurs a "break-through" of cells.

To sum up, cell-free sampling generally faces severe filtration problems thus whole culture sampling would be preferred. However, whole culture sampling is also faced with fundamental problems in that a) rapid and effective quenching of the sample must be provided, and in that b) it is more difficult to keep the transport system clean. Consequently, according to the state-of-the-art in case of whole culture sampling manual techniques are being used.

### SUMMARY OF THE INVENTION

The objective problem underlying the present invention is therefore to provide a device for automated bioreactor sampling as well as a process for automated sampling of a bioreactor. The bioreactor may contain a cell culture or a microbial fermentation. It may be used on timescales up to several weeks or months even or more. The device naturally shall be connected to a reactor and comprises a sampler, at least one tube fluidly connecting said reactor with said sampler and a pump for pumping fluids from said reactor to said sampler and vice versa.

The above-mentioned state of the art cannot provide an easy and economical sampling method for cell cultures and microbial fermentations, as such sampling is normally carried out manually and is correspondingly labour-intensive and error prone.

The present invention solves the above problem by providing means for flushing at least parts of the tubing with gas between or during individual sampling steps. The gas used is preferentially sterile air which may furthermore be dried e.g. by means of a wet separator. Depending on the culture in the reactor, it may also be nitrogen or some other gas like methane appropriate in the particular situation, while also then, these gases are preferably sterile and/or dry. Said means can be realised by some pipe or flexible tube connected with said tube, which on the one side is connected to a source of gas (e.g. sterile air) and on the other side is connected to the tube between the reactor and the sampler, and which pipe or tube can be controlled by a valve.

The object of the present invention is therefore a device according to claim 1, as well as a process according to claim 10.

One key feature of the invention is therefore that due to the flushing of at least parts of the tube with gas, i.e. for example sterile air, dead volume can be effectively minimised, which is particularly important when using automated sampling. The excess fluid in the tubing can be sent back either to the reactor or into a sample tube or alternatively into a waste receptacle. Furthermore, the use of sterile air avoids contamination of the tubing and dries it. Compared to manual sampling, the above automated sampling provides the following advantages: more consistent sample interval (automatically controlled), always fully identical taking of the sample, reduced risk of contamination due to human error, increased safety and economical benefit. Additionally, the flushing of the tubing by a gas like sterile air allows to use long tubing without having to accept higher dead volumes. In particular in laboratories with restricted space, or if such a device is being used for sampling of various different bioreactors, tubes as long as 2 m or more between the sampler and the reactor are possible without detrimental effects.

In a first preferred embodiment of the present invention, said means are flushing the tube such that fluids in the tube are pushed back into the reactor after the sampling and/or that said means are flushing the tube such that fluids in the tube are pushed into the sampler for finishing the sampling. Since appropriate gas like sterile air is being used for flushing, pushing parts of the fluids back into the reactor does not detrimentally influence the reactions in the bioreactor. Nevertheless, dead volume can be almost completely avoided. It can also be avoided, that fluids that might be stored in these tubes between individual sampling steps undergo further reaction/development which is different from the one in the bioreactor thus leading to a distortion of the results. Optionally it is possible to flush the contents of the tube not back into the reactor but completely into a waste receptacle in case contamination of the reactor might be a problem.

According to another preferred embodiment of the invention, the pump is connected with or integrated into the tube fluidly connecting said reactor with said sampler. Correspondingly, the pump is actually sucking the fluids, which may be a fermentation broth, out of the reactor, and surprisingly, this process is even possible for highly viscous fluids and does not influence the broth. Preferentially at least one of the tubes is a flexible tube, preferably a silicone tube. Using flexible tubes substantially facilitates autoclaving of the device, even more so if for control pinch valves are used with these tubes. A particularly controlled way of transporting fluids in such a system can be achieved by using peristaltic pumps, which on the one hand transport fluids but on the other hand also act like a valve and prevent flux of gas or liquid if not operating.

According to another preferred embodiment, there is provided a biomass breaker, which generally stands for some kind of (sterile) trap (e.g. sterilisable closed funnel), which on its upper part is connected with the reactor by a first tube and which on its bottom is connected with the sampler by a second tube. Thus fluid entering from the reactor drops in by gravity and fluid in the biomass breaker flows out by gravity in the direction of the sampler. Preferentially, the flushing air is also introduced into the system in the region of the biomass breaker, namely in the upper part of the biomass breaker. Preferentially the second tube is a flexible tube and the pump is a peristaltic pump acting upon said flexible tube. This set-up of the pump relative to the biomass breaker (i.e. downstream of the biomass-breaker in sampling direction) proves to be very effective and reduces dead volume problems while keeping sterility at high levels.

Further improvements of the system are possible, if means are provided for introducing rinse solution (for increasing sterility in particular in case of long studies or in case of studies prone to contaminations, e.g. animal cell cultures) and/or inactivator solution (to stop or quench the processes in the fluid prior to deposition in the sample tubes) into at least parts of the ducts between individual sampling steps. Preferentially said solution is introduced into the system in the biomass breaker, thus allowing in particular in case of an inactivator solution an effective mixing of the fluids with the inactivator solution. Optionally, there is provided at least one rinse/inactivator solution container connected with the biomass breaker by means of flexible tubes, wherein the flow of the solution is effected by a peristaltic pump. Preferably the flow is controlled by means of pinch valves (with the above-mentioned advantages), and optionally the peristaltic pump for the rinse/inactivator solution is driven by the same shaft as the peristaltic pump for the fluids (thus necessitating only one motor and one control, and assuring equal dosage). In case of using only one motor for the two pumps the provision of a bypass maybe advisable for recirculating the solution when it shall not be mixed with the fluids and to avoid unnecessary loss of the solution.

Automation can be further increased by providing a waste receptacle for taking up undesired fluids, and in that parts of the tube can automatically be shifted from the sampler to this waste receptacle.

It is optionally possible to provide a sampler, i.e. a fraction collector, allowing controlled cooling of the sample containers for storing and preferably also quenching the reaction in the sample, wherein preferentially the sample containers are located in a solid block of material such as aluminium (thus allowing effective eduction of heat) which can be cooled directly or indirectly by a cooling fluid provided by a flow cooler.

As concerns the gas like sterile air, a sterile filter, preferably with a pore diameter in the range of 0.2 µm, and/or a wet separator for removing oil and/or water present in the gas are provided in the piping for the gas for flushing.

As already mentioned, the invention also relates to a process for sampling of a bioreactor using a device as described above, preferably for cell-comprising sampling. Such a process, which is particularly suited for cell cultures and long experiments, is preferentially characterised by the following steps:

In a first step, the resting state between sampling, a robotic arm is positioned above drain that leads to a waste receptacle while at least the tube between reactor and biomass breaker is empty while the tube between reactor and drain is either empty or filled with rinse solution. In a second step the peristaltic pump is turned on, preferably for about 10 seconds, to empty the tube of rinse solution, the pump is subsequently turned off while the robotic arm moves to position above the next empty test tube in the sampler, the pump is subsequently turned on again for a time period necessary to fill the tube with extract taken from the reactor, the robotic arm subsequently returns to position above drain once pump turns off. In a third step, the pinch valves are activated opening air and rinse solution tubing, while closing bypass tubing, such that gas like sterile air pushes sample broth back into reactor and into biomass breaker tube, while as soon as the pump is turned on, sterile rinse solution enters biomass breaker and travels through tubing to waste receptacle. In a fourth step the peristaltic pump is turned off, while pinch valves remain activated for a short time period, preferably in the range of 10 to 20 seconds, to confirm that tubing from biomass breaker to reactor is empty, while as soon as the pump is turned off, the air flow rate through this tubing is augmented, the pinch valves are then deactivated to block air and rinse solution tubing.

A slightly different preferentially process, which is particularly suited for monitoring of microbial fermentation and rather shorter processes, is characterised by the following steps: In a first step, the resting state between sampling, a robotic arm is positioned above drain that leads to a waste receptacle while at least the tube between reactor and biomass breaker is empty while the tube between reactor and drain is either empty or filled with rinse solution. In a second step, the robotic arm moves to position above the next empty test tube in the sampler, the pump is subsequently turned on for a time period necessary to fill the tube, preferably between 30-90 seconds, and if desired, an inactivator solution like perchloric acid or methanol is pumped simultaneously and mixed with sample in biomass breaker. In a third step the pinch valves are activated opening gas (like sterile air) and bypass tubing, while closing inactivator solution tubing such that sterile air pushes sample broth back into reactor and into biomass breaker, with the pump on, air pushes remainder of sample/inactivator solution through tubing and into sample tube. In a fourth step the peristaltic pump is turned off and the robotic arm returns to position above drain the pinch valves remain activated for a short time period preferably in the range of 10-20 seconds, to confirm that tubing from biomass breaker to reactor is empty, while as soon as the pump is turned off, the gasflow rate through this tubing is augmented and the pinch valves are then deactivated to block air tubing.

A particular improvement of the process can be achieved in that the fluid is kept in the sampler at a temperature below 2 °C and above freezing temperature, preferably at a temperature in the range of 0.1 °C. In particular the latter proved to be very efficient in quenching typical processes in such a broth, stabilizing it for at least 24 hours, and sometimes even up to several days. Particularly efficient quenching as possible if the cooling in the sampler is effected quickly, for example if fluid can be cooled down to temperatures below 2 °C within less than 20 minutes, preferably down to temperatures below 1 °C within about 10 minutes.

Further embodiments of the present invention are outlined in the dependent claims.

### SHORT DESCRIPTION OF THE FIGURES

In the accompanying drawings preferred embodiments of the invention are shown in which:
- Figure 1: is a schematic drawing of the layout of the device for automated bioreactor sampling;
- Figure 2: is a schematic view of the individual steps for sampling of a cell culture, wherein a)-d) refer to the steps one to four as outlined in the description; and
- Figure 3: is a schematic view of the individual steps for sampling of a microbial fermentation, wherein a)-d) refer to the steps one to four as outlined in the description.

### DETAILED DESCRIPTION OF THE PREFERRED EMBODIMENTS

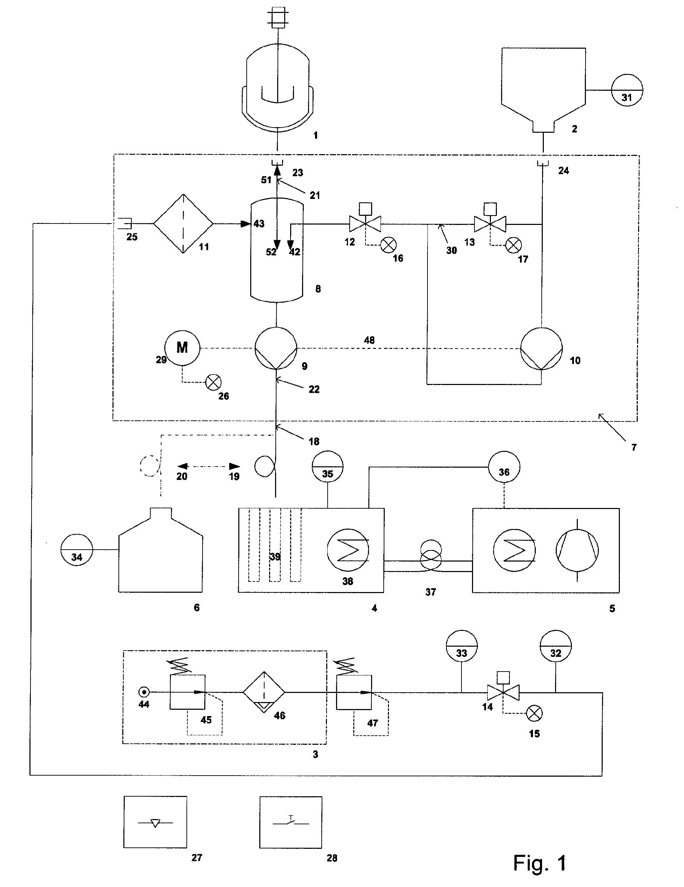

Referring to the drawings, which are for the purpose of illustrating the present preferred embodiments of the invention and not for the purpose of limiting the same, figure 1 shows a schematic drawing of a device for automated bioreactor sampling. The principal elements are the actual reactor 1, in which the reaction takes place, i.e. in which either a cell culture or a microbial fermentation process or the like is taking place. The second principal element of the device is the so-called fluidics plate 7, which governs the conduction, mixture and distribution of the different flows during and between the sampling steps. The third principal element is the sampler 4 in which the sample tubes 39 are held. The fourth principal element is given by the pressurised air unit 3 which serves to flush parts of the tubing of the fluidics plate 7.

Apart from these principal elements there is provided a container 2 in which either a rinse solution (for example sterile saline solution) or an inactivator solution (for example perchloric acid, methanol) is stored. Furthermore, a waste receptacle 6 is provided, which takes up any liquid, be it actual sampling solution or rinse solution or inactivator solution, which shall be disposed of. Both containers 2 and 6 comprise level sensors 31 and 34, respectively. The level sensor 31 of the container 2 alerts if the level of the solution drops below a certain minimal value, while the level sensor 34 alerts if the level of the waste solution exceeds a certain maximum value. The container 2 can be connected with the fluidics plate by means of a sterile coupling 24. The waste receptacle 6 can be filled by the outlet 18 of the fluidics plate 7, which by means of a robotics element can be moved between a position 20 above the waste receptacle and a position 19 above the sampler 4, or more precisely above the sample tube 39 to be filled.

Furthermore there is provided a button 28 which can be used in case of emergency (emergency stop). It brings the system immediately to a safe state (safety for operator and for reactor and samples), stops all motors and robotic elements at once and closes all the valves on the fluidics plate 7 and those forming part of the air supply. Another separate element is an alert sign 27, which indicates if the protective cover which is put on top of the fluidics plate 7 or maybe also on top of the sampler is opened or removed. Upon opening and removal of the cover, the system immediately goes into a safe state and pauses all action.

The fluidics plate 7 comprises at least 3 couplings, the first coupling being the above-mentioned sterile coupling 24 which allows to connect the tubing of the fluidics plate 7 to the container 2. A second sterile coupling 23 is provided to connect the tubing of the fluidics plate to the reactor 1, and a third coupling 25 is provided to connect the fluidics plate with the air supply. As already mentioned, the outlet 18 of the fluidics plate 7 can be automatically controlled to be positioned either above the waste receptacle 6 or above the sampler 4.

The fluidics plate 7 comprises a biomass breaker 8, which is basically a sterilizable closed funnel trap (e.g. made of glass) with three inlets on the top part and one outlet on the bottom. It is designed as a sterile trap, to the bottom of which the tube leading to the peristaltic pump 9 is connected, such that any liquid being present in the biomass breaker 8 exits via this tube. On the top part, there is one inlet 43 for sterile air. Sterile air is fed from the coupling 25 via a sterile filter 11 to this inlet 43. The sterile filter 11 can be a filter with a pore size of 0.2 µm, as for example available from PALL Medical under the trade name Pharm Assure.

The inlet 52 for fluid taken out of the reactor is directly connected via a tube 21 to the sterile coupling 23. The tube 21, as most of the tubing on the fluidics plate, is given by a silicone tube which preferably can be connected to the biomass breaker 8 by simply pushing it over a corresponding muff affixed to the biomass breaker 8. The inlet 52 is preferably designed as a rigid tube which at least partially extends vertically into the interior of the biomass breaker 8. The third inlet 42, which is either used for introducing rinse solution or for introducing inactivator solution into the biomass breaker 8 is also located on the top of the biomass breaker. The tubing leading to this inlet 42 starts at the sterile coupling 24, first goes through a peristaltic pump 10 and then can be controlled via a pinch valve 12, the status 16 of which can be monitored and controlled. For certain modes of operation, a bypass 30 is necessary, which connects the tubing on the upstream side of the peristaltic pump 10 with the tubing on the downstream side of the peristaltic pump 10 upstream of the pinch valve 12. The bypass 30 can also be controlled by means of a pinch valve 13, the status 17 of which can also be monitored and controlled.

The use of pinch valves allows for all of silicone tubing in sample loop to be autoclaved either independently or attached to the reactor.

A multi-channel peristaltic pump 9 (MiniClick2-E/5, Ismatec SA, Glattbrug-Zürich, Switzerland) is used in the tubing between the outlet of the biomass breaker 8 and the outlet 18 of the fluidics plate to pump liquid from the reactor 1 to the sampler 4. A second channel 10 pumps liquid from rinse (or inactivation solution) bottle 2 to the biomass breaker 8. The pump channels 9 and 10 are driven by a motor 29, the status 26 of which can be monitored and controlled. The sampler 4, pump channels 9 and 10, fluidics plate 7, and control computer (not displayed) are all mounted on a mobile cart.

The sampler 4 is based upon a FC203B Gilson fraction collector (Gilson SA, Villiers-le-Bel, France). It holds a single test tube rack which handles 44 tubes 39 of 14 or 15ml each. Cooling liquid maintains the samples at a given temperature by means of heat exchange coils 38 and a cooling fluid pumped via piping 37 between the cooler 5 and the sampler 4. A flow-cooler/cryostat 5 (DLK 1002, Fryka, Germany) was used to pump 50% ethylene glycol / 50% water solution at 0.1 °C through the cooling loops inside the rack. A temperature below freezing may also be maintained, although in such case excessive condensation of water from the surrounding atmosphere is provoked. The cryostat 5 is provided with a temperature sensor 36 and so is the actual sampler 4 (temperature sensor 35). It has to be noted that storing the samples at this temperature of in the range of 0.1 °C, i.e. for example between 0.05 and 0.2°C is advantageous as such and independent of the above-mentioned fluidics plate 7 in that it maximises the rate of cooling and minimises metabolic activity. Due to the high salt content even at temperatures so close to the freezing point the risk of freezing and the associated problems when thawing the samples is very limited.

The air supply 3 comprises a source of air 44 with a manual control valve 45; it additionally comprises a wet separator 46 for removing e.g. oil and water. Downstream of this pressurised air unit 3, which provides air in the pressure range up to 1 bar or even 3 bar overpressure, there is a second manual control valve 47, and further downstream there is located a first pressure sensor 33. Then follows a blow off valve 14, the status 15 of which can be controlled and monitored. Downstream of this valve 14 there is a second pressure sensor 32, downstream of which the tubing of the air unit is connected to the coupling 25 of the fluidics plate 7.

The sampler 4 is controlled through an in-house designed program written in LabVIEW (National Instruments, Austin, Texas). Through a computer interface, complete control of the sampler 4 as well as of the fluidics plate 7, the cryostat 5 and the pressurized air unit 3 is possible: e.g. opening and closing of valves, activation of pump, robotic arm movements, instrument reset, beep sound etc.

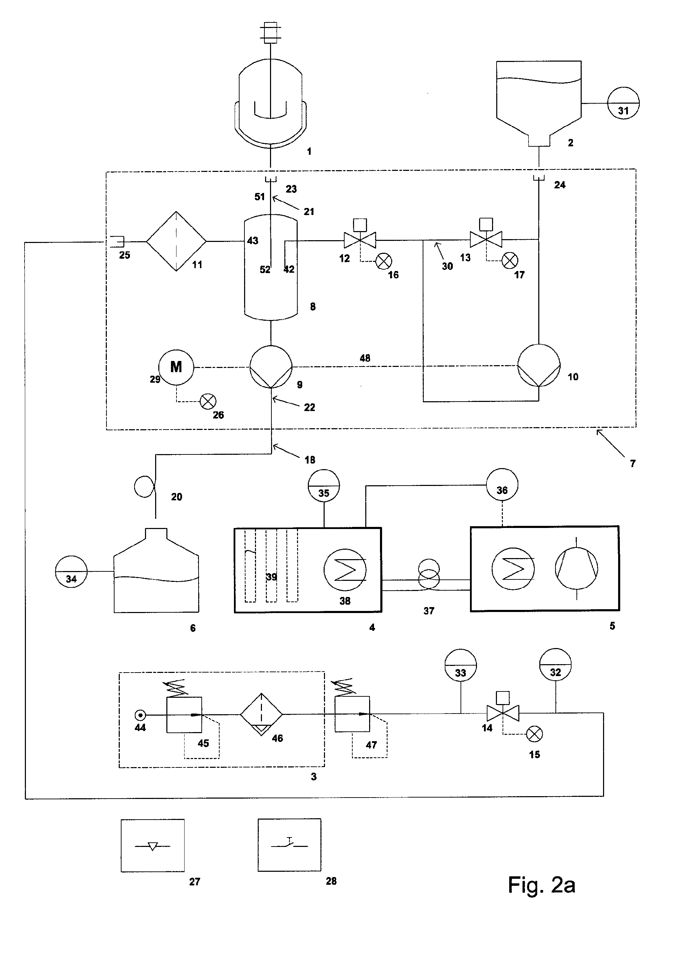

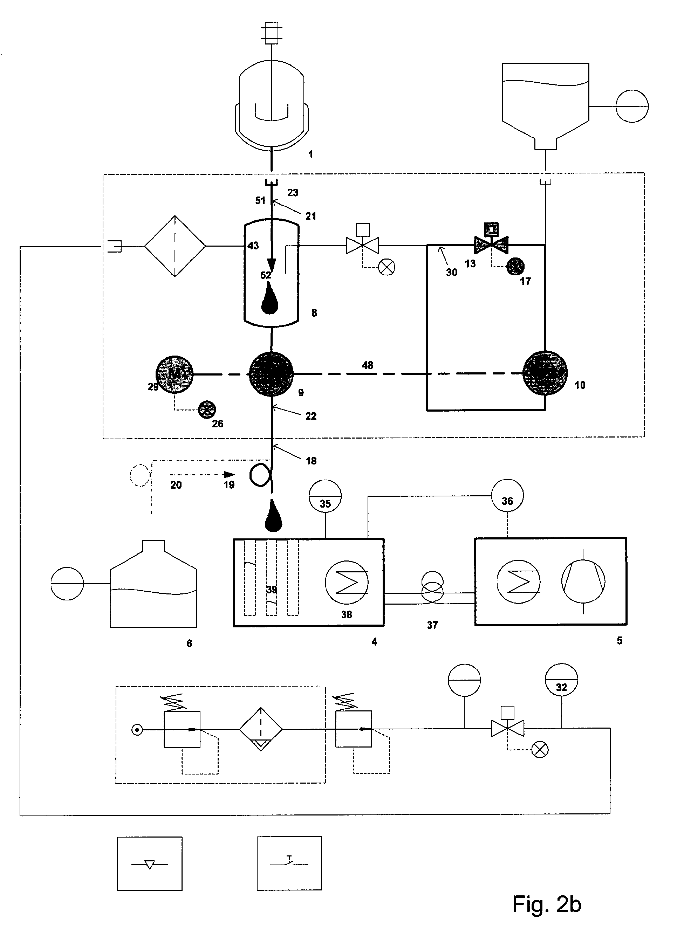

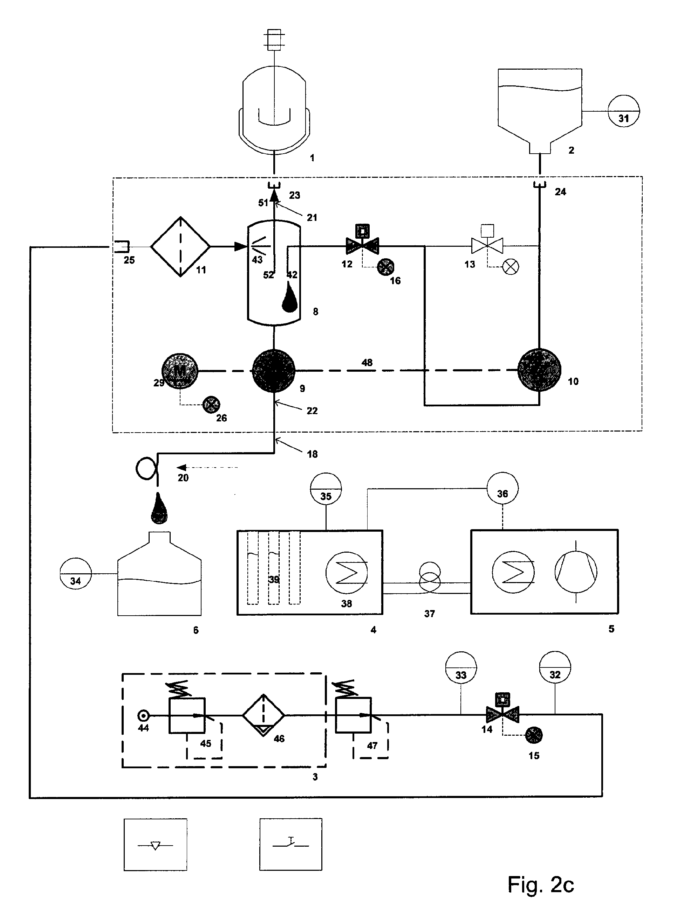

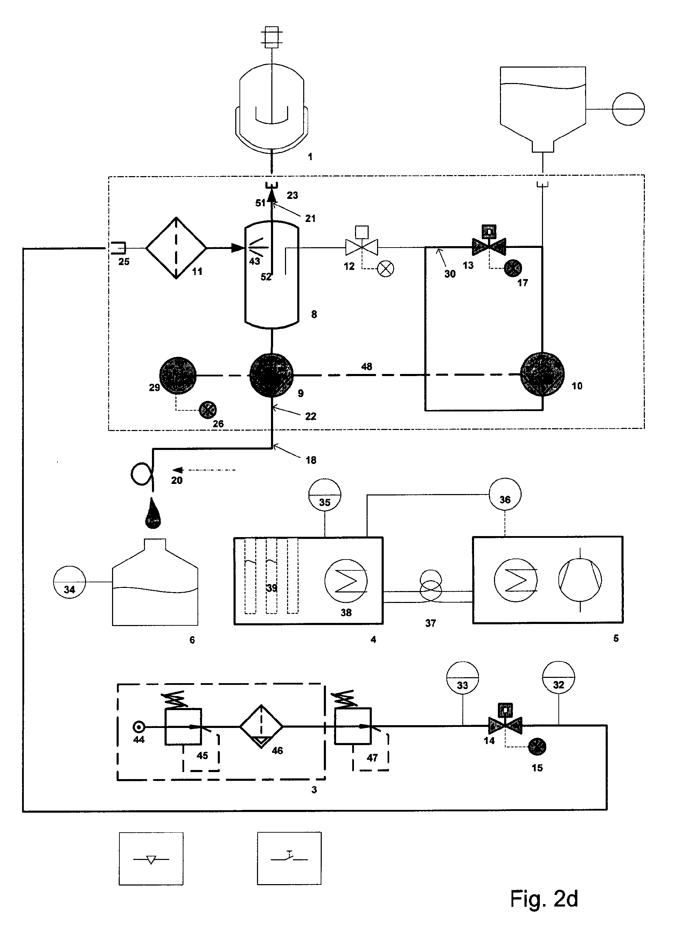

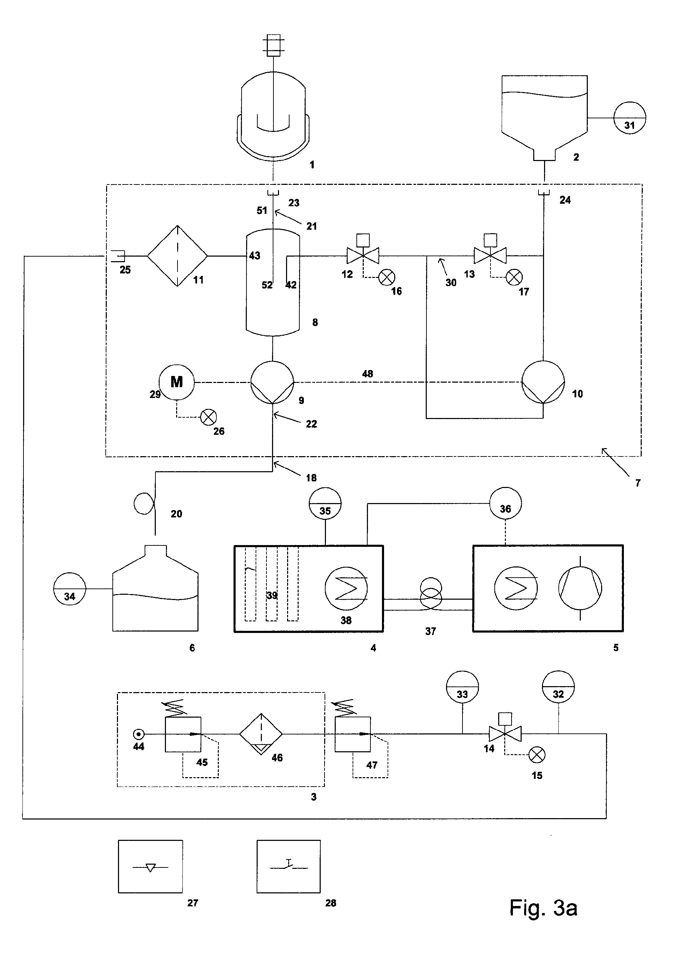

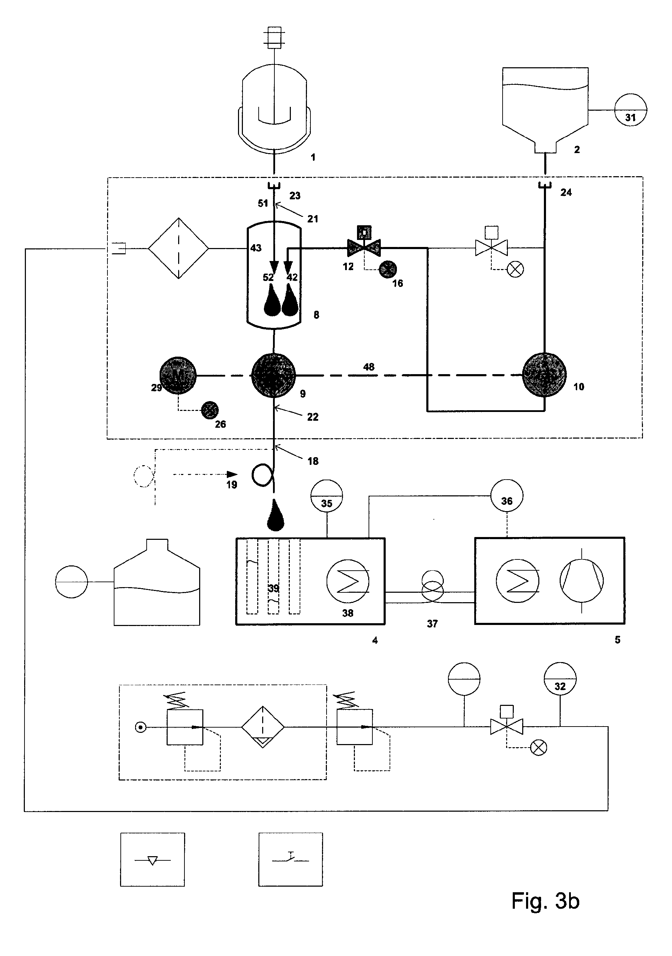

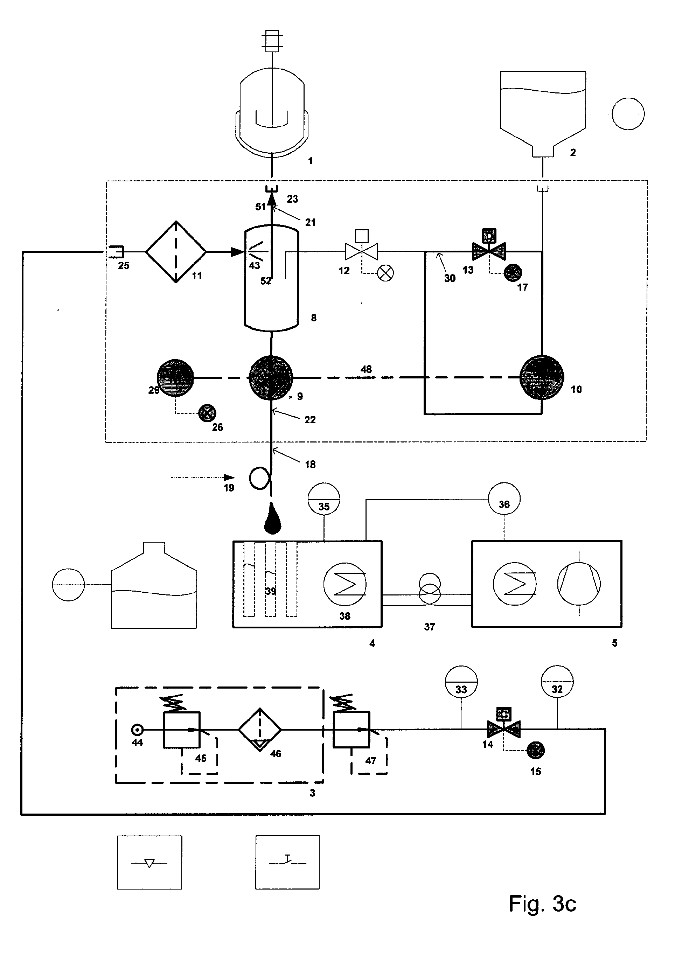

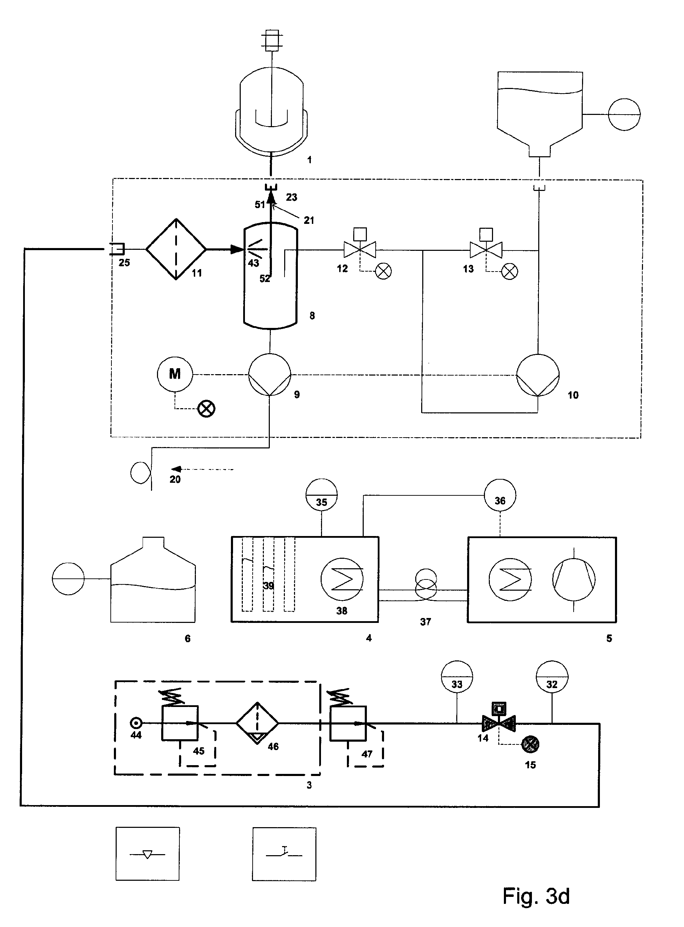

In the following, two different modes of operation shall be described in more detail. First sampling of cell cultures is described based on figure 2. Such experiments usually take rather long (typically 3-6 weeks), and since rich media are used, rinsing of the tubes is advisable. Secondly, sampling of microbial fermentation is described based on figure 3. Since such experiments are generally of shorter duration, rinsing is not that much critical and flushing with air is often sufficient.

**Cell culture sampling** (figure 2, open valves are shaded and channels through which fluid or solution is flowing are highlighted):
- *Step one* (see figure 2a):: Resting state in between samples. Robotic arm is positioned above drain that leads to waste receptacle 6. Silicone tubing 21 is empty up to biomass breaker 8. From breaker 8 to robotic arm, tubing 22 is filled with rinse solution (sterile saline).
- *Step two* (see figure 2b):: Peristaltic pump 9,10 is turned on for 10 seconds to empty tubing of rinse solution. Pump 9,10 is then turned off while robotic arm moves to position above empty test tube 39. Pump 9,10 is turned on again for time period necessary to fill test tube 39 with cell broth from reactor (30-90 seconds). Robotic arm returns to position above drain 6 once pump 9,10 turns off. The use of a single multi-channel pump 9,10 necessitates that rinse solution is circulated through the bypass 30.
- *Step three* (see figure 2c):: The pinch valves are activated, opening air 14 and rinse solution 12 tubing, while closing bypass 13 tubing. Sterile air pushes sample broth out of tubing 21 back into reactor 1. Once pump 9,10 is turned on, sterile rinse solution enters breaker 8 and travels through tubing to waste receptacle 6. This step ensures that tubing remains clean and prevents microorganism growth on rich cell culture medium in tubing exposed to atmosphere.
- *Step four* (see figure 2d):: Peristaltic pump 9,10 and rinse solution 14 and bypass 13 are turned off, while air valve 14 remains activated for short time period (∼10-20 seconds) to confirm that tubing from biomass breaker 8 to reactor 1 is empty. With pump off, the air flow rate through this tubing is augmented. Air valve 14 is then deactivated.

Sampling protocol for cell culture
1. Before autoclaving of reactor 1, attach sample apparatus (i.e. silicone tubing and biomass breaker 8).
2. Label test tubes and place in cooling rack 4.
3. Place biomass breaker 8 on fluidics plate 7 and load silicone tubing into pinch valves 12, 13.
4. Connect air at 1.0 bar to sample apparatus just before sterile Pharm Assure (PALL Medical) filters 11.
5. Adjust pump on-time of sampling program such that test tube is filled with sample broth to level even with cooling rack (∼12 ml).
6. Start auto-sample program with desired sampling interval, normally between 8 and 24 hours.
7. Every 24-72 hours, take filled sample tubes for treatment according to standard protocols like centrifugation, separation of supernatant, further cooling to - 20 °C for storage and subsequent HPLC or ELISA analysis, haemocytometer count with Trypan blue, dilution in Isoton at appropriate dilution for Coulter Counter analysis, etc.
8. Samples stored at 0.1 °C in cooling rack are stable for up to 100 hours.

**Microbial fermentation sampling** (figure 3, open valves are shaded and channels through which fluid or solution is flowing are highlighted)
- *Step one* (see figure 3a):: Resting state in between samples. Robotic arm is positioned above drain that leads to waste receptacle 6. All tubing, from reactor 1 to robotic arm, is empty.
- *Step two* (see figure 3b):: Robotic arm moves to position above empty test tube 39. Pump 9,10 is turned on for time period necessary to fill tube with broth from the reactor (30-90 seconds). If desired, an inactivator solution 2 (e.g. perchloric acid) is pumped simultaneously and mixed with sample in biomass breaker 8.
- *Step three* (see figure 3c):: The pinch valves are activated which opens air 14 and bypass 13 tubing, while closing inactivator solution 12 tubing. Sterile air pushes sample broth back into reactor 1 and into biomass breaker tube 8. With pump 9,10 on, air pushes remainder of sample/inactivator solution 2 through tubing 22 and into sample tube 39. Thus, there is no dead volume for the sample collection procedure. The use of a single multi-channel pump necessitates that inactivator solution 2 is circulated through bypass 30 tubing.
- *Step four* (see figure 3d):: Peristaltic pump 9,10 and inactivator 12 and bypass 13 are turned off and robotic arm returns to position above drain 6. Pinch valve 14 remains activated for short time period (∼10-20 seconds) to confirm that tubing 21 from biomass breaker 8 to reactor 1 is empty. With pump 9,10 off, the airflow rate through tubing 21 is augmented. Pinch valve 14 is then deactivated to block air.

Sampling protocol for microbial fermentations
1. Before autoclaving of reactor 1, attach sample apparatus (i.e. silicone tubing and biomass breaker 8). For reactors sterilized in situ, sample apparatus may be sterilized independently and then connected sterilely in same manner as feed and/or base connections are made.
2. Place tubes in defined order in cooling rack of sampler 4.
3. Place biomass breaker 8 on fluidics plate 7 and load silicone tubing into pinch valves 12, 13.
4. Connect air at 1.5 bar to sample apparatus just before sterile Pharm Assure (PALL Medical) filters 11.
5. Adjust pump on-time of sampling program such that test tube is filled with samplebroth to level even with cooling rack (∼12 ml).
6. Start autosample program with desired sampling interval, normally between 0.25 and 4 hours.
7. Every 12-48 hours, transfer filled sample tubes directly to centrifuge at 4 °C and subject them to subsequent standard off-line analyses.

### Experimental Results

### Cooling rate of samples at initial temperatures of 22 °C, 30 °C, and 37 °C

In order to validate the system, the rate at which samples were cooled down was measured. The sample tubes were first placed in the cooling rack cooled to 0.1 °C and allowed to equilibrate. In center of tube, an RTD (PT1OO) temperature probe was fixed at a height of 0, 2, 4, or 6 cm. At time zero, 12 ml deionized water at 22, 30, or 37 °C was added. The temperature probe, which was at an initial temperature of 0.1 °C, was not quick enough to record initial temperature, but rather only reached a value 5-10 °C below initial water temperature. Quite clearly the water lower in the tube was cooled more quickly. However, the temperature was below 5 °C in all cases, regardless of starting temperature or probe height, within 10 minutes. The temperature at, or very near, to bottom of tube remained at zero, or evenly slightly below 0 °C. With cells present, they settled very rapidly to bottom of tube, and were hence stored at such a low temperature. There is a 0.5 mm gap between sample tube and wall of rack, which slows down heat transfer. However, due to condensation, a film of water was present which dramatically improved the transfer of heat. In fact, for the measurements at 22 °C, 0.5 ml of water was added to space between rack and sample tube one hour before measurement, and in this case, even the water 6 cm from bottom of tube was below 5 °C in 3.5 minutes. For the measurements at 30 and 37 °C, the residual water from condensation in rack was not controlled. However, the cooling rate was noticeably inferior to that at 22 °C.

### CRD perfusion cell culture

To compare results from manual sampling with the proposed method, seven samples from CHO perfusion culture were taken at the same time and placed in the cooling rack at 0.1 °C. One sample was immediately analysed, while the other samples were analysed individually over a period of 140 hours. No significant variation was seen in glucose or lactate measurements for any of the samples. The cellular viability was constant for at least 100 hours, but the sample analysed at 140 hours indicated that viability has started to decrease. The mean cell diameter remained constant for the duration of experiment.

Once it was clear that storage of the samples at 0.1 °C for an extended period of time did not alter results, the device was utilized to collect samples from several CHO perfusion culture experiments. The samples were collected every eight hours for over 400 hours. Manual sampling at such a high rate would have required a tremendous effort. One advantage of a high sampling rate is that rates can be easily calculated and trends become apparent that otherwise would have been missed.

### Microbial fermentations

The proposed device has been successfully used to sample numerous batch, fed-batch, and continuous fermentations of Saccharomyces cerevisiae, Phaffia rhodozyma, and Pichia pastoris yeast and Acetobacter xylinus bacteria. With rapid sampling provided by the device and automated off-line HPLC analysis, the culture dynamics are easily followed. Glycerol and ethanol profiles were dearly defined even though their concentration never exceeded 400 mg/l.

In another application, samples were collected from high cell density S. cerevisiae fed-batch cultures. The dry cell weight data was accurate to within 0.1 g/l, even for cell densities up to 100 g/l. Furthermore, it was possible to quantify concentrations below 100 mg/l of most of the TCA cycle metabolites. The concentration profiles increased slowly, until the onset of nitrogen starvation phase at 14.4 h, at which point concentrations increased dramatically. In a subsequent experiment with no nitrogen starvation, the concentrations remained much lower, i.e. 1 g/l. These results indicate that the low temperature of sample tubes was sufficient to arrest metabolite activity and an inactivator solution was not required. However if it were needed, then the proposed device is configured such that an inactivator solution 2 (e.g. percholoric acid or methanol) could be rapidly mixed with sample in biomass breaker tube 8. If a syringe pump were used to add solution, then the volume could be precisely controlled. Alternatively, the volume of inactivator added could be quantified during HPLC analysis cell broth.

Herein, a novel method for automated bioreactor sampling is described, and then applied to animal cell culture and microbial cell fermentations. Standardised sample collection reduces the risk of contamination and simplifies subsequent data analysis since data spacing is uniform. By following detailed sampling protocol, the time involved in monitoring a microbial cultivation is significantly reduced since samples are treated in parallel. Rapid cooling of samples to 0.1 °C was sufficient to stop all metabolic activity. However, at higher temperatures (2-4 °C), this was not the case. Thus, it sometimes might be critical that samples are kept at or very near to 0.0 °C. Although samples could have been frozen with the system, this was deemed to be undesirable, as surely some lysis would occur upon thawing of cells. Furthermore, condensation was a much greater problem with temperatures below 0.0 °C.

The proposed system is inexpensive and simple to operate. It is of great utility to any process that requires frequent sampling done in a reproducible manner.

### LIST OF REFERENCE NUMERALS

- 1: reactor
- 2: rinse solution/inactivator solution
- 3: pressurised air unit
- 4: sampler
- 5: flow cooler
- 6: waste receptacle
- 7: fluidics plate
- 8: biomass breaker
- 9: peristaltic pump (medium)
- 10: peristaltic pump (rinse)
- 11: sterile filter
- 12: pinch valve (rinse/inactivator)
- 13: pinch valve (bypass)
- 14: blow-off valve
- 15: status of 14
- 16: status of 12
- 17: status of 13
- 18: outlet of the fluidics plate
- 19: position of 18 above sampler
- 20: position of 18 above waste receptacle
- 21: tubing
- 22: tubing
- 23: sterile coupling (to reactor)
- 24: sterile coupling (to rinse solution)
- 25: coupling (to pressurised air unit)
- 26: status of 29
- 27: protective cover open/closed
- 28: emergency stop
- 29: motor of peristaltic pump
- 30: bypass
- 31: level sensor
- 32: pressure sensor
- 33: pressure sensor
- 34: level sensor
- 35: temperature sensor
- 36: temperature sensor
- 37: piping
- 38: heat exchange coils in 4
- 39: sample tubes
- 42: inlet rinse solution into biomass breaker
- 43: inlet pressurised air into biomass breaker
- 44: source/inlet of pressurised air
- 45: manual control valve
- 46: wet separator
- 47: manual control valve
- 48: common shaft to 9 and 10
- 51: inlet sterile air/broth into reactor
- 52: inlet reactor solution into biomass breaker

## Claims

1. Device for automated bioreactor sampling to be connected to a reactor (1), comprising, a sampler (4), at least one tube (21,22) fluidly connecting said reactor (1) with said sampler (4) and a pump (9) for pumping fluids from said reactor (1) to said sampler (4) and vice versa,
**characterized in that**
means (3,14) are provided for flushing at least parts of the tube (21,22) with gas between individual sampling steps.

2. Device according to claim 1, **characterised in that** said gas is flushing the tube (21) such that fluids in the tube (21) are pushed back into the reactor (1) after the sampling and/or that said gas is flushing the tube (22) such that fluids in the tube (22) are pushed into the sampler (4) for finishing the sampling.

3. Device according to any of the preceding claims, **characterised in that** the pump (9) is connected with or integrated into the tube (21, 22) fluidly connecting said reactor (1) with said sampler (4), wherein preferentially at least one of the tubes (21, 22) is a flexible tube, preferably a silicone tube, and wherein even more preferentially the pump (9) is a peristaltic pump.

4. Device according to claim 3, **characterised in that** there is provided a biomass breaker (8) which on its upper part is connected with the reactor (1) by a first tube (21) and which on the bottom is connected with the sampler (4) by a second tube (22), wherein the flushing gas is introduced into the system on the upper part of the biomass breaker (8), and wherein preferentially the second tube (22) is a flexible tube and the pump (9) is a peristaltic pump acting upon said flexible tube (22).

5. Device according to claim 4, **characterised in that** means (2) are provided for introducing rinse solution and/or inactivator solution into at least parts of the tubes (21, 22) between individual sampling steps, wherein preferentially said solution is introduced into the system in the biomass breaker (8).

6. Device according to claim 5, **characterised in that** there is provided at least one rinse/inactivator solution container (2) connected with the biomass breaker (8) by means of flexible tubes, wherein the flow of the solution is effected by a peristaltic pump (10) and preferably the flow is controlled by means of pinch valves (12, 13), and wherein preferentially the peristaltic pump (10) for the rinse/inactivator solution is driven by the same shaft (48) as the peristaltic pump (9) for the samples, and wherein even more preferentially a bypass (30) is provided for recirculating the solution when it shall not be mixed with the samples.

7. Device according to any of the preceding claims, **characterised in that** there is provided a waste receptacle (6) for taking up undesired fluids, and **in that** parts of the tube (22) can automatically be shifted from the sampler (4) to this waste receptacle (6).

8. Device according to any of the preceding claims, **characterised in that** the sampler (4) is a fraction collector allowing controlled cooling of the sample containers (39), wherein preferentially the sample containers (39) are partially embedded in a block of material such as aluminium which can be cooled directly or indirectly by a cooling fluid provided by a flow cooler (5) which cooling fluid is preferentially circulated through internal channels of the block.

9. Device according to any of the preceding claims, **characterised in that** a sterile filter (11), preferably with a pore diameter in the range of 0.2 µm, and/or a wet separator (46) are provided in the piping for the air for flushing.

10. Process for sampling of a bioreactor using a device according to one of the claims 1 to 9, preferably for cell-comprising sampling.

11. Process according to claim 10, **characterised in that**
in a first step, the resting state between sampling, a robotic arm is positioned above drain that leads to a waste receptacle (6) while at least the tube (21) between reactor (1) and biomass breaker (8) is empty while the tube (22) between reactor (1) and drain is either empty or filled with rinse solution,
in a second step the peristaltic pump (9,10) is turned on, preferably for about 10 seconds, to empty the tube (22) of rinse solution, the pump (9,10) is subsequently turned off while the robotic arm moves to position above the next empty test tube (39) in the sampler (4), the pump (9,10) is subsequently turned on again for a time period necessary to fill the tube (39) with sample/broth from the reactor, the robotic arm subsequently returns to position above drain once pump (9,10) turns off,
in a third step, the pinch valves (12-14) are activated opening gas/air (14) and rinse solution (12) tubing, while closing bypass (13) tubing, such that sterile air pushes sample broth back into reactor (1), while as soon as the pump (9,10) is turned on, sterile rinse solution (12) enters biomass breaker (8) and travels through tubing to waste receptacle (6), and
in a fourth step the peristaltic pump (9,10) and rinse solution (14) and bypass (13) are turned off, while gas/air valve (14) remains activated for a short time period, preferably in the range of 10 to 20 seconds, to confirm that tubing from biomass breaker (8) to reactor (1) is empty, while as soon as the pump (9,10) is turned off, the air flow rate through this tubing is augmented, the gas/air valve (14) is then deactivated to block gas/air.

12. Process according to claim 10, **characterised in that**
in a first step, the resting state between sampling, a robotic arm is positioned above drain that leads to a waste receptacle (6) while at least the tube (21) between reactor (1) and biomass breaker (8) is empty while the tube (22) between reactor (1) and drain is either empty or filled with rinse solution,
in a second step, the robotic arm moves to position above the next empty test tube (39) in the sampler (4), the pump (9,10) is subsequently turned on for a time period necessary to fill the test tube (39) with sample/broth from the reactor (1), preferably between 30-90 seconds, and if desired, an inactivator solution like perchloric acid is pumped simultaneously and mixed with sample in biomass breaker (8),
in a third step the pinch valves (12-14) are activated opening air (14) and bypass (13) tubing, while closing inactivator solution (12) tubing such that sterile air pushes sample broth back into reactor (1), with the pump (9,10) on, air pushes remainder of sample/inactivator solution through tubing and into sample tube (39),
in a fourth step the peristaltic pump (9,10) and inactivator (12) and bypass (13) are turned off and the robotic arm returns to position above drain (6) the gas/air valve (14) remains activated for a short time period preferably in the range of 10-20 seconds, to confirm that tubing from biomass breaker (8) to reactor (1) is empty, while as soon as the pump (9,10) is turned off, the airflow rate through this tubing is augmented and the gas/air valve (14) is then deactivated to block gas/air.

13. Process according to one of the claims 10-12, **characterised in that** the fluid is kept in the sampler (8) at a temperature below 2 °C and above freezing temperature, preferably at a temperature in the range of 0.1 °C.

14. Process according to claim 13, **characterised in that** fluid can be cooled down to temperatures below 2 °C within < 20 minutes, preferably down to temperatures below 1 °C within about 10 minutes.
